# EUROPEAN PATENT APPLICATION

(11) **EP 2 727 621 A1**
(43) Date of publication of application: **07.05.2014**
(21) Application number: 13189432.1
(22) Date of filing: 18.10.2013
(51) Int. Cl.: A61M 25/02, A61M 25/04

(54) **Bartholin gland drainage catheter**

(30) Priority: 01.11.2012 US 201261721141 P; 03.10.2013 US 201314045335
(71) Applicant: Cook Medical Technologies LLC, Bloomington, IN 47404 (US)
(72) Inventor: Duncan, Kate, Mooresville, IN Indiana 46158 (US); Woodard, Bryan D., Bloomington, IN Indiana 47401 (US)
(74) Representative: Garratt, Peter Douglas

(57) **Abstract**

A Bartholin gland drainage catheter, a method of using the drainage catheter, and a kit containing a drainage catheter are disclosed. The drainage catheter comprises a shaft having an external diameter, a proximal end and a distal end and a drainage lumen extending between the respective ends. The distal end of the shaft comprises a retention mechanism, such as a Malecot, having a plurality of expandable wings which anchor the catheter within the Bartholin gland. The wings are selectively moveable from a first radially expanded position to a second radially compressed insertion position. The proximal end of the catheter shaft comprises an anchoring element having an external diameter that is greater than the external diameter of the shaft which remains outside of the patient's labial tissue. The catheter is inserted into the gland with the Malecot retained in a compressed configuration, and then subsequently allowed to expand within the gland, thus securing the catheter in place to facilitate drainage and healing of a cystic gland.

## Description

### Technical Field

The present invention relates generally to catheters and more specifically, to a catheter for draining a body cavity including the Bartholin gland.

There are a variety of therapies or treatments that require use of a drainage catheter, and more specifically, a drainage catheter with a distal anchor or similar structure to position and secure the catheter to or within a particular portion of a patient's body.

For example, one particular situation in which use of a drainage catheter having a distal anchor is useful and desirable involves catheters used for drainage of the Bartholin gland. In particular, women have two Bartholin glands located in the labia minora near the opening of the vagina. These glands make small amounts of fluid that moisten the vulva. On occasion, one or both of these glands can become blocked or clogged, causing a back-up of fluid which may lead to a cyst or abscess. The cysts are usually small and painless; however, they may become enlarged, inflamed and/or infected, causing much discomfort to a patient. In such a case, treatment of the infected gland may become warranted or necessary. Currently, treatment for an infected Bartholin gland may include surgically removing the gland and duct, or, alternatively, draining the gland and duct.

Accordingly, it is desirable to provide a simplified and improved catheter and methods of using the same, including but not limited to, one for the treatment and drainage of the Bartholin gland.

### Summary

A catheter adapted for drainage of the Bartholin gland is disclosed. In one example, the catheter comprises a shaft having an external diameter and a proximal and distal end and an interior drainage lumen extending between the proximal and distal ends. The distal end of the shaft comprises a retention mechanism having a plurality of expandable wings which are biased in a first radially expanded open position and are moveable to a second radially compressed insertion position. The catheter further comprises an anchoring element at the proximal end of the shaft having an external diameter that is greater than the external diameter of the shaft. The catheter further comprises a substantially rounded tip located distal to the retention mechanism.

In one example, at least a portion of each of the plurality of expandable wings are biased radially outwardly in an arcuate configuration and at least a portion of the retention mechanism has an external diameter that is greater than the external diameter of the shaft when the retention mechanism is in the first radially expanded position. The retention mechanism may comprise a Malecot.

A method for using a catheter for drainage of a Bartholin gland is also disclosed. In one example, the method comprises the steps of providing a drainage catheter comprising a shaft having a proximal end and a distal end and an interior drainage lumen extending between the proximal and distal ends, wherein the distal end of the shaft comprises an expandable retention mechanism and the proximal end of the shaft comprises an anchoring element. The method further comprises compressing the retention mechanism into a radially inwardly compressed configuration for insertion into the gland, inserting at least the distal end of the drainage catheter into the gland and positioning the retention mechanism in a desired location within the gland. The method further comprises selectively manipulating the retention mechanism from a radially inwardly compressed insertion configuration to a radially outwardly expanded configuration and positioning the anchoring element at the proximal end of the catheter shaft outside of the gland adjacent to the external labial tissue.

A kit including a drainage catheter is also disclosed. In one example, the kit comprises a device adapted for creating a tissue incision and a drainage catheter adapted for insertion into a body cavity at a selected location. The catheter may comprise a shaft having interior lumen extending between a proximal end and a distal end, wherein the distal end of the shaft comprises a retention mechanism comprising a first radially expanded open position and a second radially compressed insertion position, the retention mechanism being moveable between the first and second positions, and wherein the proximal end of the shaft comprises an anchoring element. The kit may further comprise an elongated insertion tool configured to be positioned within the lumen of the catheter shaft to facilitate movement of the catheter retention mechanism from the first expanded position to the second radially compressed configuration for insertion into a body cavity.

### Brief Description of the Drawings

Figure 1 illustrates the normal anatomy of a female patient including the Bartholin glands.

Figure 2 illustrates a cyst formed in one of the Bartholin glands.

Figure 3 is a perspective view of a drainage catheter in accordance with an embodiment of the present invention in a radially outwardly expanded configuration.

Figure 4 illustrates the distal end of a tool being used to create an opening in the labial tissue proximal to the Bartholin gland.

Figure 5 illustrates an insertion tool being used to manipulate a drainage catheter in a radially inwardly compressed configuration in accordance with an embodiment of the present invention for insertion through a tissue opening and into the Bartholin gland.

Figure 6 is a side view of one embodiment of a drainage catheter that has been inserted and positioned in the Bartholin gland.

### Detailed Description

Throughout this specification the terms proximal and proximally are used to refer to a position or direction away from, or even external to a patient's body and/or tissues, and the terms distal and distally are used for a position or direction towards the patient and/or to be inserted into a patient's body or tissues. The embodiments described below are primarily in connection with the use and insertion of an implantable medical device, such as a catheter for the treatment and drainage of a Bartholin gland cyst. However, it will be understood that the described apparatus and methods may also be used in connection with a range of implantable medical devices, such as drainage catheters for the treatment of other parts of the body and internal orifices or cavities.

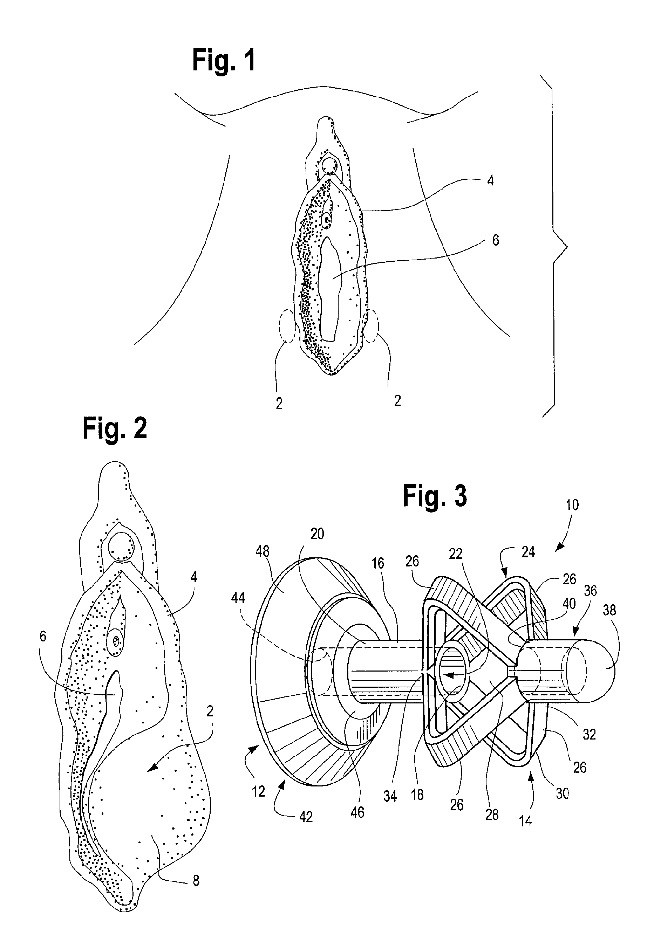

Referring to Figure 1, the anatomy of a healthy female patient, including the Bartholin glands, is shown. The Bartholin glands 2 are located in the labia minora 4, at the opening of the vagina 6. In a normal condition, the Bartholin gland(s) 2 makes small amounts of fluid which drain through a duct to moisten the vagina. However, as illustrated in Figure 2, one or both of these glands can become blocked or clogged, causing a back-up of the fluid which may lead to a cyst or abscess 8. In such a case, treatment and drainage of the cyst 8 may become warranted or necessary.

One embodiment of a device that may be used for drainage of a body cavity, including, but not necessarily limited to the Bartholin gland 2, is generally illustrated in Figure 3. Preferably, the device comprises a drainage catheter 10 that may be used for the drainage of a cyst 8 that has formed in one or both of the Bartholin glands. The catheter 10 includes a proximal end 12 which generally remains outside of the patient's body and a distal end 14 which is introduced through the patient's tissue and is anchored into and within the gland 2. In one embodiment, the catheter 10 may be made of a polymeric material, including, but not limited to silicone, polyurethane and/or other suitable materials. The material is preferably medical grade and able to withstand extended indwellment of up to several weeks. In one non-limiting example, the catheter may comprise silicone in the form of either liquid or gum-stock, with a durometer of about 50-80.

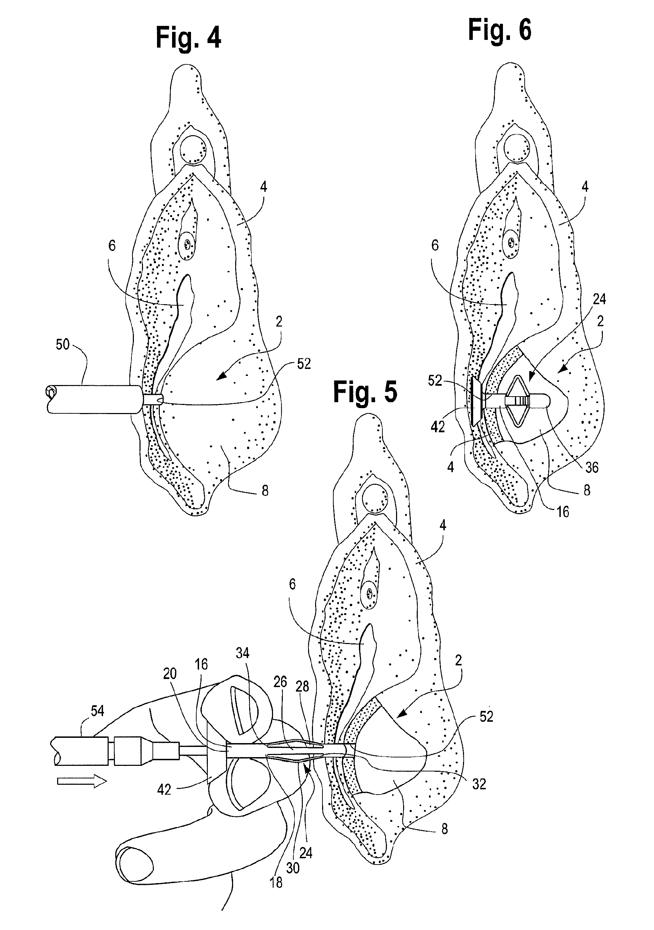

The catheter 10 illustrated in Figure 3 is shown in a radially expanded or deployed state, while Figure 5 illustrates the catheter in a radially compressed, low-profile state, designed for insertion and delivery into the body cavity or gland.

More specifically, as shown in Figure 3, the catheter 10 includes an elongated linear member or shaft 16 having proximal 20 and distal 18 ends with a drainage lumen 22 extending there between. A retention mechanism 24 is positioned near the catheter distal end 14. The retention mechanism 24 preferably includes one or more members that extend radially outwardly, thus serving as an "anchor" to hold the distal end 14 of the catheter 10 in a desired position within the gland 2. In a preferred embodiment, the retention mechanism 24. In one example, the retention mechanism 24 comprises a plurality of radially expandable wings or arms 26, and more particularly, the retention mechanism 24 may include a Malecot. However, it is also contemplated that the retention mechanism 24 can include a variety of radially outwardly expanding or expandable structures including, but not limited to posts, wings, fins, pigtail loop(s), struts, balloons and/or discs, alone or in combination with a Malecot, for retaining the catheter 10 in position within the gland 2.

As shown in Figures 3 and 5, the Malecot 24 may include a plurality of arms or wings 26 separated by a plurality of slots 28. When the Malecot 24 is in the radially compressed insertion configuration as shown in Figure 5, the plurality of arms 26 are generally arranged in parallel with the slots 28 that lie there between along the longitudinal axis of the catheter 10. The arms 26 and slots 28 are preferably about the same length.

However, the Malecot arms 26 are generally biased in a radially outwardly expanded position as illustrated generally in Figure 3 such that when the Malecot 24 is in its natural relaxed state, the arms 26 define a bent or arcuate shape, such that the circumference of the radially-outward most portion 30 of the Malecot 24 in its expanded form is substantially larger than the outer circumference of the Malecot in its compressed form. Each of the plurality of Malecot arms 26 includes a distal end 32 and a proximal end 34. The arms 26 merge together at the proximal end 34 where they meet or otherwise adjoin the distal end 18 of the catheter shaft 16. Similarly, the distal ends 32 of the respective Malecot arms 26 also merge together where they meet and adjoin a tip 36 located distal to the Malecot 24 and/or other retention mechanism.

More specifically, as shown in Figure 3, a tip 36 is located distal to the Malecot 24. In one embodiment, the tip 36 terminates at its distal end 38 in a substantially rounded, preferably smooth surface. The proximal end 40 of the tip 36 adjoins with the distal end 32 of the retention mechanism or Malecot 24, where the Malecot arms 26 merge at 32 as described above. In one embodiment, the tip 36 is a substantially solid structure, but it is also contemplated that the tip 36 may include a lumen extending partially or entirely there through.

As further illustrated in Figure 3, an external anchoring element or "bung" 42 is preferably located adjacent to the proximal end 20 of the catheter shaft 16. The bung 42 remains external to the patient's body and prevents the proximal end 20 of the catheter shaft 16 from sliding into the body cavity or gland 2. The bung 42 and the catheter shaft 16 may be a unitary structure formed or molded from the same piece of material or they may be molded separately and then secured together such as by adhesive, welding, bonding or the like. The bung 42 preferably includes an opening or aperture 44 formed in the center thereof which is generally aligned with the catheter internal lumen 22, such that any fluid draining or flowing through the lumen 22 may also pass freely through the aperture 44 in the bung 42. In one embodiment, the bung 42 is preferably disc-shaped, although, a variety of other suitable configurations and shapes are also contemplated. As Figure 3 shows, the bung 42 has a distal end portion 46 and a proximal end portion 48. The distal end portion 46 of the bung 42 is adjacent to and meets with the proximal end portion 20 of the catheter shaft.

In one embodiment, the proximal end 48 of the bung 42 extends radially outwardly from the catheter shaft 16 and flares or tapers slightly in a rearward or proximal direction, thus forming a gradual dome or truncated cone shape. In other words, the proximal end 48 or "mouth" of the bung 42 which defines the widest portion of the taper faces in the proximal direction away from the catheter shaft 16. In other embodiments, the bung 42 may be substantially flat (i.e., such that it is not tapered and/or not flared) or alternatively, the bung may flare or taper in the opposite direction such that the widest portion or "mouth" 48 of the bung faces the distal end 14 of the catheter. Preferably, the bung 42 is shaped such that it will lie substantially flat against the external tissue of the labia as shown in Figure 6, and as a result, will not get caught or snagged on clothing or otherwise become dislodged due to patient movement or activity or rubbing by clothing or surrounding tissue.

An exemplary method of delivering and deploying an implantable medical device such as the drainage catheter described herein is now provided. In one embodiment of the disclosed method and as shown in Figure 4, a physician will preferably create a small incision in the patient's labial tissue 4. This may serve to lance a cyst 8 that has formed in the Bartholin gland 2, allowing some fluid from cyst 8 to be initially drained and subsequently, provides an opening for the drainage catheter 10 to be inserted for placement within the gland 2. This tissue opening may be made by various known methods, such as by a scalpel, a laser and preferably, by a punch biopsy tool 50 as illustrated in Figure 4. In one example, a 4 mm or similarly sized punch biopsy tool 50 may be used so that there will be a consistently sized hole or opening 52 in the tissue, however a scalpel or other methods may also be used to create variable hole sizes. Depending on the size of the incision that is ultimately made, the opening may, if desired, be sutured to ensure that a catheter 10 that has been placed in the gland 2 is retained in its proper position.

Once the tissue opening 52 has been created, the catheter 10 is deliverable to a target site, such as the Bartholin gland, in several ways. As mentioned above, it is preferable to manipulate the retaining mechanism or Malecot 24 into a radially compressed configuration for insertion through the small incision or opening 52 in the patient's labial tissue and into the gland. Straightening the catheter 10 into a low-profile configuration, in which the retaining mechanism 24 is manipulated or otherwise deformed into a radially compressed configuration has various advantages including increasing ease of insertion as well as patient comfort. As Figure 5 illustrates, it is preferable to compress the wings 26 of the Malecot 24 such that the external diameter or outer circumference of the outermost portion 30 of the Malecot wings 26 is reduced to become substantially the same as the external diameter of the catheter shaft 16. The Malecot 24 may be compressed into this "insertion configuration" in several ways.

In one example, the physician may simply compress the wings 26 of the Malecot 24 manually, such as by depressing the wings with their fingers during insertion. Once the Malecot has been inserted through the tissue opening 52 and the distal end 14 of the catheter placed in a desired location, the physician can simply release the Malecot 24 from their fingers. Because the Malecot wings 26 are biased radially outwardly, the retaining mechanism will return to the expanded configuration within the gland 2 as the Malecot wings 26 assume their natural expanded arcuate shape as shown in Figures 3 and 6.

Alternatively, the physician can use a tool for insertion and delivery of the catheter 10. In one example, the physician may use a separately provided stylet, obturator or similar linear "stiffening" tool 54 to straighten the catheter 10 into a streamlined, low-profile configuration designed for easy and comfortable insertion through the patient's tissue and placement within the gland 2. As Figure 5 shows, the distal end or tip of the obturator 54 is placed through the aperture 44 in the bung 42 and into the lumen 22 of the catheter and pushed forward (i.e. in a distal direction) while holding the bung 42 and/or catheter proximal end 12 stationary. This causes the catheter 10 to extend slightly (longitudinally), such that the Malecot wings 26 are substantially straightened and become radially inwardly compressed. As such, the device is configured in a streamlined configuration for insertion through the tissue opening 52. When the distal end 14 of the catheter is properly positioned within the gland 2, the physician may remove the obturator 54 from the catheter lumen 22, thus allowing the Malecot wings 26 to naturally return to the radially outwardly expanded position due to their inherent elasticity and resiliency. As illustrated in Figure 6, the expanded Malecot 24 anchors the distal end 14 of the catheter 10 within the gland 2 while the bung 42 remains outside of the patient's body and lies snugly against the external labial tissue 4. This arrangement holds the catheter 10 in position securely, where it can remain in place for extended periods of time, as necessary.

It is contemplated that the catheter 10 remains in place for several days and/or up to several weeks. Preferably, the catheter is designed to withstand indwellement of at least 29 days, and in some instances, the catheter may remain in place up to six weeks. As Figure 6 shows, the catheter shaft 16 holds open the tissue planes of the labia 4, Bartholin gland 2 and duct, allowing the cyst 8 to drain and heal. While some fluid may drain around the external surface of the catheter, the internal lumen 22 of the catheter provides a drainage pathway while the catheter 10 is in place. This added drainage may help prevent the gland 2 from becoming blocked or infected again, while also allowing the gland to return to its normal function of secretion if the gland heals before the catheter is removed. If desired, the stylet or obturator 54 may also be used to straighten the catheter 10 into a radially compressed streamlined configuration for convenient and comfortable removal of the catheter as best illustrated in Figure 5. Alternatively, a physician may pull gently on the proximal end 12 of the catheter 10 and/or on the bung 42 with their fingers, such that the force of the surrounding tissue presses the Malecot wings 26 radially inward to allow manual removal of the catheter 10 from the healed gland.

The above-described drainage catheter 10 may be sold and/or provided to healthcare providers and physicians either alone or in the form of a kit or packaged set. In one example, the drainage catheter 10 may be provided in a kit with one or more other tools or devices that are used during the treatment and drainage of a Bartholin gland cyst 8. Such a kit may preferably include a drainage catheter 10 as described herein, a tool 50 for creating a tissue incision and an obturator 54 or stylet to facilitate insertion and delivery of the catheter 10 through the tissue incision 52 and into the gland 2. Of course, the kit may also contain more or fewer tools than those previously described as required or necessary for the particular procedure being performed.

In addition to the various advantages mentioned above, the catheter 10 described herein achieves several additional advantages over prior devices including, but not limited to the fact that it is substantially smaller in size and shorter in length relative to balloon catheters because an inflation lumen is not needed. The relatively short catheter length, flat/low profile of the bung and the structure used to anchor the catheter in a desired position not only improve the appearance, practicality and convenience of the device but also improve overall patient comfort. A patient can resume normal activity with minimal restrictions while the catheter indwells for extended periods of time with less risk of the catheter becoming dislodged and eliminating the need and inconvenience of an additional re-insertion procedure.

Throughout this specification, unless the context requires otherwise, the words "comprise" and "include" and variations such as "comprising" and "including" will be understood to imply the inclusion of an item or group of items, but not the exclusion of any other item or group items.

While various embodiments of the invention have been described, it will be apparent to those of ordinary skill in the art that many more embodiments and implementations are possible within the scope of the invention. Furthermore, although various indications have been given as to the scope of this invention, the invention is not limited to any one of these but may reside in two or more of these combined together. Accordingly, the invention is not to be restricted except in light of the attached claims and their equivalents.

## Claims

1. A drainage catheter comprising:
a shaft having an external diameter and a proximal and distal end,
an interior drainage lumen extending between the proximal and distal ends,
wherein the distal end of the shaft comprises a retention mechanism having a plurality of expandable wings, the wings being biased in a first radially expanded open position and moveable to a second radially compressed insertion position, and
wherein the proximal end of the shaft comprises an anchoring element having an external diameter that is greater than the external diameter of the shaft;
the catheter further comprising a substantially rounded tip located distal to the retention mechanism.

2. The catheter of claim 1 wherein the anchoring element is substantially disc-shaped.

3. The catheter of claim 1 or claim 2 wherein at least a portion of each of the plurality of expandable wings are biased radially outwardly in an arcuate configuration.

4. The catheter of any one of the previous claims wherein at least a portion of the retention mechanism has an external diameter that is greater than the external diameter of the shaft when the retention mechanism is in the first radially expanded position.

5. The catheter of claim 4 wherein the retention mechanism comprises a Malecot.

6. The catheter of any one of the previous claims wherein the interior drainage lumen is configured to receive a distal tip of an obturator pressed against a portion of the catheter distal tip, wherein the force applied to the catheter distal tip longitudinally elongates the catheter, thus moving the expandable wings of the retention mechanism from the radially expanded configuration to the radially compressed position.

7. The catheter of any one of the previous claims wherein at least a portion of the catheter comprises a polymeric material.

8. The catheter of claim 7 wherein the polymeric material comprises silicone.

9. The catheter of any one of the previous claims wherein the catheter is a single unitary molded component.

10. The catheter of any one of claim 1 to claim 8 wherein the catheter comprises at least two separately molded components bonded together.

11. The catheter according to any one of the preceding claims adapted for use in a method for drainage of a Bartholin gland comprising the steps of:
a. providing a drainage catheter comprising a shaft having a proximal end and a distal end and an interior drainage lumen extending between the proximal and distal ends, wherein the distal end of the shaft comprises an expandable retention mechanism and the proximal end of the shaft comprises an anchoring element;
b. compressing the retention mechanism into a radially inwardly compressed configuration for insertion into the gland;
c. inserting at least the distal end of the drainage catheter into the gland,
d. positioning the retention mechanism in a desired location within the gland;
e. manipulating the retention mechanism from a radially inwardly compressed insertion configuration to a radially outwardly expanded configuration;
f. positioning the anchoring element at the proximal end of the catheter shaft outside of the gland adjacent to the external labial tissue.

12. A kit comprising:
a. a device adapted for creating a tissue incision;
b. a drainage catheter adapted for insertion into a body cavity at a selected location, the catheter comprising a shaft having interior lumen extending between a proximal end and a distal end, wherein the distal end of the shaft comprises a retention mechanism comprising a first radially expanded open position and a second radially compressed insertion position, the retention mechanism being moveable between the first and second positions, and wherein the proximal end of the shaft comprises an anchoring element;
c. an elongated insertion tool configured to be positioned within the lumen of the catheter shaft to facilitate movement of the catheter retention mechanism from the first expanded position to the second radially compressed configuration for insertion into a body cavity.

13. The kit of claim 12 wherein the device for creating a tissue incision comprises at least one of a scalpel, a laser and a punch biopsy tool.

14. The kit of claim 12 or claim 13 wherein the retention mechanism comprises a Malecot.
